# EUROPÄISCHE PATENTSCHRIFT

(11) **EP 2 104 519 B1**
(45) Veröffentlichungstag und Bekanntmachung des Hinweises auf die Patenterteilung: **14.12.2011**
(21) Anmeldenummer: 07818856.2
(22) Anmeldetag: 09.10.2007
(51) Int. Cl.: A61L 26/00, A61K 8/04, A61K 9/70, A61K 47/06

(54) **FILMBILDENDE GELKOMPOSITION ZUR WUND- BZW. HAUPTPFLEGE**
FILM-FORMING GEL COMPOSITION FOR WOUND OR SKIN CARE
COMPOSITION DE GEL FILMOGÈNE POUR SOIGNER LA PEAU OU UNE BLESSURE

(30) Priorität: 19.10.2006 DE 102006049929
(43) Veröffentlichungstag der Anmeldung: 30.09.2009
(73) Patentinhaber: Beiersdorf AG, 20253 Hamburg (DE)
(72) Erfinder: WÖLLER, Karl-Heinz, 20257 Hamburg (DE); NIERLE, Jens, 21147 Hamburg (DE)
(86) Internationale Anmeldenummer: PCT/EP2007/008783
(87) Internationale Veröffentlichungsnummer: WO 2008/046541

(56) Entgegenhaltungen:
- WO-A-98/23291
- WO-A-99/27014
- WO-A-2006/065991
- US-A- 5 622 711
- US-A1- 2004 018 250

## Beschreibung

Die Erfindung umfasst eine, bevorzugt kosmetische und/oder dermatologische, filmbildende Zubereitung umfassend mindestens ein hydrophobes Gel und mindestens ein hydrophiles Gel.

Halbfeste Zubereitungen in Form von Salben, Cremes und Gelen zur Wundbehandlung sind weltweit verbreitet und bilden das Standardvehikel um eine Wunde mit einem medizinischen Wirkstoff zu versorgen. Hierbei wird der Wirkstoff direkt und konzentriert an den Wirkort verbracht, wobei die Wirkdauer lediglich von der jeweiligen Wechselwirkung zwischen Wirkort und Substanz abhängig ist. Nachteilig ist, dass eine blutende oder stark sezernierende Wunde die innere Struktur der Zubereitung dabei leicht zerstört und die Wirkstoffe wieder ausspülen kann.

Gelegentlich werden die Wirkstoffe zur Wundbehandlung auch in Form flüssiger Zubereitungen wie Lösungen oder Lotionen auf das zu behandelnde Hautareal verbracht. Hierbei ist der Effekt des Ausspülens noch stärker zu beobachten bzw. die flüssige Zubereitung verläuft schon bei der Applikation über nicht zur Behandlung vorgesehene Hautareale.

Höhere Akzeptanz finden diese flüssigen oder halbfesten Zubereitungen zur Versorgung gesunder Haut mit kosmetischen Wirkstoffen.

Eine andere Form halbfester Zubereitungen stellen pflasterartige Matrixsysteme dar, bei denen der Wirkstoff entweder in die klebende Matrix inkorporiert ist und direkt oder aus einem Reservoir durch die klebende Matrix hindurch an den Wirkort penetriert. Pflastermatrices haben gegenüber den oben beschriebenen flüssigen oder cremeartigen Zubereitungen den Vorteil, dass die Wirkstofffreisetzung über einen längeren Zeitraum erfolgt und gesteuert werden kann.

Nachteilig ist, dass sich Pflaster aufgrund der vorgegebenen Form an vielen Wirkorten nicht optimal anpassen lassen bzw. die Wirksubstanz nicht nur an den vorgesehenen Wirkort appliziert wird sondern auch auf Körperflächen, die nur zur sicheren Fixierung der Pflaster benötigt werden.

Generell nachteilig zu eigen ist allen halbfesten Zubereitungen, dass sich lediglich Wirkstoffe mit analoger Polarität der Creme- bzw. Klebmatrix in größeren Mengen einarbeiten lassen und diese dann aufgrund der vorhandenen Löslichkeit nur schwer bei der Anwendung wieder aus den Matrices freigesetzt werden.

Bei Zubereitungen in Form von Salben, Cremes und Gelen werden daher üblicherweise entsprechende Mengen an Lösungsvermittlern und/oder Penetrationsbeschleunigern zugesetzt. Bei Zubereitungen in Pflasterform kommen zur Behebung dieses Nachteils ebenfalls häufig Lösungsvermittler/Penetrationsbeschleuniger zum Einsatz. Ebenfalls können zusätzlich Hydrokolloide zur Penetrationsverbesserung eingearbeitet werden. Diese Hydrokolloide können beschränkte Mengen an Wasser oder wässrigen Lösungen aufnehmen. Bei größeren Mengen an Flüssigkeit wird die grundlegende Matrixstruktur allerdings in ihren Produkteigenschaften häufig negativ beeinflusst.

US 20040191279 A1 beschreibt ein lipophiles Matrixsystem auf Polyisobutylenbasis dem eine nicht weiter definierte Menge an Wasser zugesetzt werden kann. Diese Matrixsysteme können zur Verbesserung der Klebkraft zwischen der menschlichen Haut und Einmal-Hygieneartikeln wie Windeln, Damenbinden, kosmetischen Pflastern etc. eingesetzt werden, welche zur Aufnahme der unterschiedlichen Körperexsudate auf der Haut appliziert werden. Um ein Fließen dieser Matrixsysteme zu verhindern, werden immobilisierende Agenzien wie Wachse und Fettsäure(-poly-)ester eingesetzt. Der Schmelzpunkt dieser Matrixsysteme liegt dabei zwischen 44 und 104 °C.

Ein weiteres klebendes Matrixsystem zur Feuchtigkeitsaufnahme, vorzugsweise bei Ostomie-Anwendungen, ist in der US 6303700 B1 beschrieben. Zur schnelleren Exsudataufnahmefähigkeit des grundlegenden Polyisobutylensystems wird dabei der Zusatz von Polyvinylpyrrolidon bzw. Polyvinylpyrrolidon Vinylacetat Copolymer dargelegt.

In der EP 724888 A2 sind hydrokolloidale Matrices beschrieben und insbesondere ein System zur Wundbehandlung, bei dem das Exsudataufnahmevermögen einer wässrigen Lösung eines hydrophilen Blockpolymers mittels der Gel-Sol Umwandlungstemperatur eingestellt werden kann. In einem Bereich von 0 - 40 °C, vorzugsweise im Bereich einer üblichen Wundtemperatur von 30 - 40 °C, wird dabei das wasserlösliche Sol in das wasserunlösliche Gel umgewandelt.

US 20040018250 A1 beschreibt eine Öl-in-Wasser Wachs Dispersion für topische und analoge Anwendungen als Lotion, Salbe, Paste, Serum oder Spray. Hierbei wird ein Wachs wie z.B. Kakaobutter, Vaseline etc. mit einer oder mehreren hydrophoben Komponente wie z. B. hydrierte Rizinus- oder Palmkernöle als hydrophobe Phase vermengt und diese hydrophobe Phase mit einer wässrigen Phase unter hoher Scherung / hohem Druck zur Öl-in-Wasser Wachs Dispersion vermengt. Die im Homogenisator zu verwendenden Drücke zur Erzielung der beschriebenen Partikelgröße der Dispersion von 0,1 -0,5 microns betragen dabei 750 - 1850 bar.

US5622711 beschreibt eine Klebmasse die eine ungesättigtes aliphatisches, hydrophobes Homopolymer, einen Tackifier und ein Hydrokolloid enthält. Als Tackifier wird u.a. Polyisobutylen erwähnt, als Hydrokolloid werden u.a. Polyacrylate genannt. Das Hydrophobe Polymer kann ein Gel ausbilden, und der Gesamtzubereitung können darüber hinaus Mineralöle zugesetzt werden.

WO2006/065991 offenbart eine Öl-in-Wasser Dispersion mit dispergierter Phase enthaltend Wirkstoff, und Polymer oder Lipid, sowie eine gelierte kontinuierliche Phase.

WO99/27014 beschreibt eine klebende Hydrokolloidkomposition umfassend Polyisobutylen, hydrokolloidales Pulver, pyrogene Kieselsäure, Gummi, Styrolcopolymere, Klebrigmacher, Mineralöle und wahlweise antimikrobielle Mittel.

Wünschenswert wäre es demnach eine Zubereitung zur Verfügung zu stellen, die die Nachteile der im Stand der Technik bekannten halbfesten Kompositionen, wie Cremes oder Lotion, als auch die Nachteile der Pflasterapplikationen nicht aufweist, dabei aber die Vorteile der beiden Wund- bzw. Hautbehandlungsformen kombiniert.

Vorteilhaft ist es auch eine alternative Pflege bzw. Behandlungsform zu den bekannten halbfesten Zubereitungen zur Verfügung zu stellen.

Die Erfindung umfasst eine, bevorzugt kosmetische und/oder dermatologische, filmbildende Zubereitung umfassend mindestens ein hydrophobes Gel und mindestens ein hydrophiles Gel. Das hydrophobe Gel wird aus mindestens einem hydrophoben Polymer und einem oder mehreren Mineralfetten und/oder -ölen und ggf. sonstigen, üblichen Zusatzstoffen, gebildet.

Eine solche erfindungsgemäße Zubereitung, insbesondere in Gelform, umfasst eine homogene Mischung des hydrophoben Polymergels mit einem hydrophilen Polymergel, einem Hydrogel.

Aus dem Stand der Technik ist bekannt, dass Polymere in organischen Lösemitteln quellen und sich dann nach und nach auflösen. Fette und Öle können Polymere ebenso quellen und lösen, so dass eine Kombination aus hydrophoben Polymeren und Fetten oder Ölen bislang zu instabilen Mischungen geraten. Beispielsweise wird Polypropylen langsam durch mittelkettige Triglyceride aufgelöst.

Ebenso bekannt ist, dass Fette und Öle als Weichmacher für Polymere verwendet werden. Umfassen hydrophobe Polymere aber mehr als 50 Gew.% dieser Weichmacher, bezogen auf die Gesamtmasse, wird normalerweise der Strukturverbund der Polymere untereinander aufgelöst.

Überraschend hat sich nun gezeigt, dass sich hydrophobe Polymere mit Mineralfetten oder - ölen zu einem stabilen Gel formulieren lassen ohne dass das Gel die nachteiligen Auflösungserscheinungen aufweist.

So bildet beispielsweise Polyisobutylen (PIB) in Paraffinöl und/oder Vaseline eine stabile, je nach Anteil der öligen Bestandteile, weiche innere Gelstruktur aus. Polyisobutylen quilt dabei mit Paraffinölen/Vaseline zu einer gelartigen Struktur, die auch bei PIB-Anteilen unterhalb von 50 Gew.% ausreichend Kohäsivität besitzt, um bei Handhabung weich und formbar zu sein und zudem nicht in einzelne Bestandteile zu zerfallen.

Bevorzugt ist daher das hydrophobe Polymer zu einem Anteil von maximal 60 Gew.%, bevorzugt maximal 50 Gew.%, insbesondere maximal 40 Gew.%, bezogen auf die Gesamtmasse des sich bildenden Gels, im Gel enthalten ist.

Der Anteil an Mineralfetten bzw. ölen ist somit entsprechend hoch wählbar, so dass auch stabile Gele erhalten werden, wenn der Mineralfettanteil 40 oder 50 Gew.% und mehr beträgt. Der Anteil ist hier jeweils bezogen auf die Gesamtmasse des sich bildenden hydrophoben Gels.

Erfindungsgemäß bevorzugte hydrophobe Polymere sind Polyisobutylene, insbesondere
Hochmolekulares PIB:
   Polyisobutylen mit einem gewichtsmittleren Molekulargewicht (M_{w}) von 300.000 bis 1.100.000, bevorzugt zwischen 650.000 und 850.000. Solche Polymere sind kommerziell beispielsweise unter den Handelsnamen Oppanol B100 (BASF) oder Oppanol B80 (BASF) erhältlich.
Niedermolekulares PIB:
   Polyisobutylen mit einem gewichtsmittleren Molekulargewicht (M_{w}) von 40.000 bis 300.000, bevorzugt zwischen 60.000 und 100.000. Solche Polymere sind kommerziell beispielsweise unter den Handelsnamen Oppanol B15 (BASF) erhältlich.

Das erfindungsgemäße hydrophobe Polymergel umfasst bevorzugt zwischen 10 und 60 Gew.% thermoplastisches Polymer, bevorzugt Polyisobutylen. Besonders bevorzugt enthält das hydrophobe Polymergel 15 bis 45 % PIB, ganz besonders bevorzugt 20 bis 40 % PIB. Den Rest des hydrophoben Gels bilden bevorzugt die aufgeführten Mineralfette und/oder - öle, wie Vaseline und/oder Paraffinöl, aber auch weitere hydrophobe Zusätze wie z.B. Wachse, Bienenwachs, zur Einstellung der Geschmeidigkeit des Endprodukts, hydrophobe Wirkstoffe, Lösungsvermittler/Penetrationsbeschleuniger für die Wirkstoffe und/oder Hilfs-, Zusatz oder Wirkstoffe.

Erfindungsgemäß ist das hydrophobe Gel ein formbeständiges, leicht deformierbares disperses System bestehend aus einem lipophil quellbaren Polymer (z.B. PIB, SIS) und einem oder mehreren Mineralfetten und/oder -ölen als Dispersionsmittel. Dabei ist die feste Substanz kohärent, d.h. sie bildet im Dispersionsmittel ein räumliches Netzwerk. Die komplexe dynamische Viskosität solcher lipophilen PIB-Gele kann beispielsweise im Bereich von 10 000 - 25 000 Pa*s (25° C; 1 rad/s) liegen.

Bevorzugt wird als hydrophobes Polymer Polyisobutylen gewählt. Weiter bevorzugt wird Paraffin(öl) und/oder Vaseline als Mineralfette bzw. -öle gewählt.

Insbesondere Paraffinöl und Vaseline werden als bevorzugte Mineralfette gewählt, insbesondere in einem Verhältnis von Vaseline zu Paraffinöl von 1 : 5 bis 5 : 1, bezogen auf deren Gewichtsanteile im hydrophoben Gel. Bevorzugt ist ein Verhältnis von ca. 1:1.

Wird erfindungsgemäßes hydrophobes Gel hergestellt, so lässt sich dieses mit einem oder mehreren hydrophilen Gelen, z.B. Hydrokolloidgelen, zu einer streichfähigen aber nicht fließfähigen und insbesondere filmbildenden Gelkomposition kombinieren.

Dabei liegen die hydrophilen Bestandteile innerhalb der hydrophoben Gele homogen verteilt vor. Das hydrophile Gel bildet dabei diskrete Tropfen aus, die mit einer durchschnittlichen Grössen von ca. 0,2 - 20 µm innerhalb des hydrophoben Gels verteilt sind.

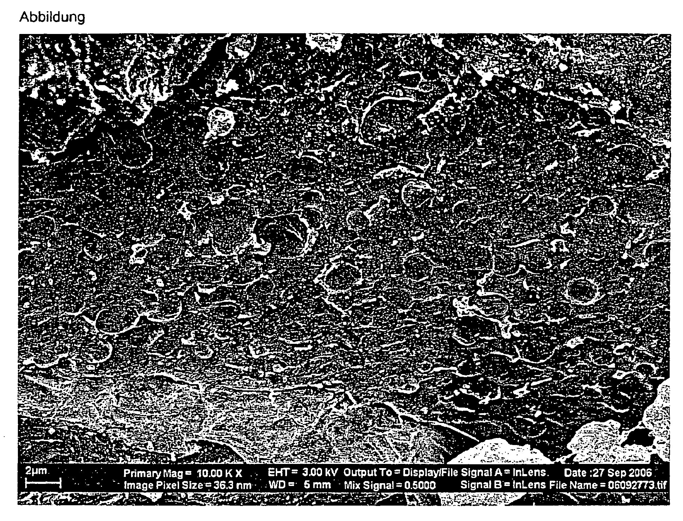

In Abbildung 1 ist beispielhaft eine REM-Aufnahme einer erfindungsgemäßen Gelkomposition, Muster WC 06/001, dargestellt. Nach Gefrierpräparation stellen dabei die kreisförmigen Abbildungen die Verteilung der hydrophilen Geltröpfchen innerhalb des lipophilen Gels dar. Die Abbildung wurde wie folgt präpariert:
Probenpräparation: Einfrieren in flüssigem Stickstoff (-196 °C), mechanischer Bruch der Probe, Gefrierätzung bei -130 bis -100 °C und 1,5 * 10⁻⁷ mbar, Bedampfung mit Wolfram.

Die filmbildenden finalen Gelkompositionen bestehend aus lipophilen und hydrophilen Gelen können je nach Zusammensetzung als Endprodukt komplexe dynamische Viskositäten ausbilden, die sich signifikant von den dynamischen Viskositäten eines reinen lipophilen respektive hydrophilen Gels unterscheiden.

| Probe | Komplexe dynamische Viskosität 25 °C; 1 rad / s |
|---|---|
| | Pa*s |
| Hydrophiles Gel | 1 710 |
| Lipophiles Gel | 17 860 |
| erfindungsgemäße Gelkomposition 1 | 55 980 |
| erfindungsgemäße Gelkomposition 2 | 11 350 |

| | |
|---|---|
| (Meßsystem: Frequenztest: Platte / Platte 25 mm, 1 mm Spalt) | |

Die resultierende erfindungsgemäße Zubereitung, insbesondere eine Gelkomposition, lässt sich anschließend wie eine Einreibung auf dem betroffenen Hautareal verteilen und bildet dann mit der Zeit einen festen Film aus, der sich, wie ein Pflaster, als Ganzes wieder abziehen lässt. Diese einmalige Art und Weise - Auftragung wie eine Creme, Haut- und Wundbehandlung wie Creme oder Pflaster und Entfernen wie ein Pflaster - kombiniert in innovativer Art die Vorteile der halbfesten Kompositionen, wie Cremes oder Lotion, als auch der Pflasterapplikationen.

Die Zeit zur Filmbildung steht dabei in Abhängigkeit vom jeweiligen Wassergehalt der Gelkomposition, den gewählten Polymeren und/oder der Auftragsmenge/Schichtdicke der Zubereitung auf der Haut.

Es können somit individuelle Haut- und Wundbehandlungszubereitungen zur Verfügung gestellt werden, die sich in ihrer Filmbildung den jeweiligen Ansprüchen anpassen lassen. Beispielweise wäre bei eine Wundpflegezubereitung eine Filmbildung innerhalb mehrerer Stunden bzw. Tage sinnvoll, eine kosmetische Gesichtsmaske beispielweise eher im Bereich einiger Minuten.

Vorteilhaft ist die erfindungsgemäße Zubereitung damit in der Wundbehandlung und/oder der Hautpflege einsetzbar.

Die erfindungsgemäße Zubereitung lässt sich aufgrund ihrer Streichfähigkeit auf einer Wunde aufgetragen. Dies wird erfindungsgemäß als cremeartig definiert. Aufgrund der Nicht-Fließfähigkeit verbleibt die Zubereitung auf dem Hautareal ohne abzufließen. Die von einer Wunde abgegebene Flüssigkeit, wie Blut, Sekret, Exsudat, kann von dem hydrophilen Gel in der erfindungsgemäßen Komposition aufgenommen werden. Gleichzeitig kommt es auf der hautabgewandten Seite des Gelfilms permanent zu einem Flüssigkeits-/ Wasserverlust durch Verdunstung. Die innere Struktur der Gelkomposition ist dabei so ausgebildet, dass es immer zu einem Austausch zwischen dem hydrophoben Gel in sich und dem hydrophilen Gel in sich kommt. Sobald die Wunde soweit verheilt ist und kein neues Sekret, keine Flüssigkeit mehr abgegeben wird, trocknet der hydrophile Anteil der Komposition langsam aus. Der sich bildende feste Film lässt sich dann wie ein Pflaster als Ganzes wieder abziehen.

Das Prinzip der Filmbildung ist dabei wie bei einem Sprühpflaster nur mit dem wesentlichen Anwendungsvorteil, dass sich der Film wieder von der Haut abziehen lässt und nicht mit der Haut fest verklebt bleibt. Der erfindungsgemäße sich auf der Haut bildende Film lässt sich gegenüber den bekannten Pflasterapplikationen somit vor allem schmerzfrei wieder entfernen.

Die Nicht-Fließfähigkeit wird dabei bei RT und Normaldruck konstatiert.

Weiterer Vorteil der erfindungsgemäßen Zubereitungen liegt darin, dass die Zubereitung ein feuchtes Wundmilieu aufrecht erhält. Es ist bekannt, dass eine feuchte Wundheilung schneller verläuft als unter trockenen Bedingungen.

Somit kann man ein semi-okklusives Wundmanagementsystem einstellen das aktiv eine schnelle feuchte Wundheilung fördert.

Weitere vorteilhafte Ausgestaltung der erfindungsgemäßen Zubereitung ergibt sich durch eine Auswahl von unterschiedlichen Brechungsindices der hydrophilen und hydrophoben Gele.

Beispielsweise wird das hydrophile Gel auf Basis von Polyacrylsäure in Wasser (10 Gew.% Polyacrysläure) mit einem Brechungsindex von z.B. 1,34 - 1,35 gewählt.

Das hydrophobe Gel, beispielsweise gebildet aus 35 Gew.% PIB in 29 Gew.% Vaseline und 36 Gew.% Paraffinöl (Verhältnis ca. 1 : 1,2), weist einen Brechungsindex von z.B. 1,47 - 1,49 auf.

Damit weist die Kombination der Gele, die erfindungsgemäße Zubereitung, eine creme- oder salbenartige weiße Farbe bzw. Trübung auf.

Bei Austrocknung des wässrigen Gelanteils in der Wundversorgung, wie zuvor beschrieben, verliert sich diese Färbung und der Gelfilm wird transparent, da sich der Brechungsindex im Bereich des auf der Haut verbleibenden klar-durchsichtigen hydrophoben PIB-Gels verändert.

Diese Eigenschaft kann als Indikator für die Anwendungsdauer der erfindungsgemäßen Komposition genutzt werden.

Dem Anwender kann somit anwendungsfreundlich ein Mittel zur Wundbehandlung oder - Hautpflege angeboten werden, das ihm das Ende der Wundheilung oder der Anwendungsdauer optisch anzeigt.

Neben der Anwendung zur Wundbehandlung kann die erfindungsgemäße Zubereitung vorteilhaft auch zu kosmetischen Zwecken angewendet werden, insbesondere zur Hautpflege z.B. als Gesichtsmaske.

Das Hautpflege - und/oder Wundheilungsprodukt umfassend eine erfindungsgemäße Zubereitung ist cremeartig auf der Haut verteilbar und nach der Anwendungsdauer als Film wieder entfernbar.

Durch die geschilderte Auswahl an verschiedenen Gelen mit unterschiedlichen Brechungsindices kann das Ende der Anwendungsdauer des Hautpflege- und/oder Wundheilungsproduktes durch Veränderung der Brechungsindizes optisch angezeigt werden.

Das oder die hydrophoben Polymere werden bevorzugt gewählt aus der Gruppe der Polyisobutylene (PIB), Polybutadien, Polyisopren, Styrol-Isopren-Styrol-Blockcopolymere (SIS), Styrol-Butadien-Styrol-Blockcopolymere (SBS) und Styrol-Butadien-Rubber (SBR). Zur Optimierung der finalen Produkteigenschaften können zur Herstellung des hydrophoben Gels auch Mischungen aus zwei oder mehreren der aufgeführten hydrophoben Polymere in unterschiedlichen Mengenverhältnissen verwendet werden.

Bevorzugt ist Polyisobutylen zu wählen. Als hydrophobes Polymer ist eine Mischung aus Polyisobutylen und anderen hydrophoben Polymeren, bevorzugt SIS, ebenfalls bevorzugt.

Mineralfette stehen für die aus mineralischen Rohstoffen (Erdöl, Braun- u. Steinkohlen, Holz, Torf) gewonnenen Destillationsprodukten, die im wesentlichen aus Gemischen von gesättigten Kohlenwasserstoffen bestehen. Insbesondere sind darunter die festen und halbfesten Gemische höherer Kohlenwasserstoffe zu verstehen, wie insbesondere Ceresin, Vaseline, Paraffin. Die Mineralfette umfassen erfindungsgemäß insbesondere Verbindungen gewählt aus der Gruppe der Paraffinöle- oder wachse, Weißöle, Vaseline, Ceresin, Bienenwachs etc.

Als Mineralfette oder -öle sind ein oder mehrere Fette oder Öle gewählt aus der Gruppe Paraffinöle, Paraffinfette, insbesondere Vaseline, und Paraffinwachse bevorzugt zu wählen.

Bevorzugt sind Paraffinöl und/oder Vaseline zu wählen.

Vaseline ist ein Mineralfett, dass als halbfestes Paraffin(öl) (*Petrolatum*) als gelbe *oder weiße Vaseline* im Handel erhältlich ist. Vaseline wurde erstmals von Chesebrough 1871 aus pennsylvan. Rohölen hergestellt.

Unter hydrophilen Gelen werden die in der Kosmetik bekannten Gele bevorzugt eingesetzt. Die Herstellung eines solchen hydrophilen Polymergels erfolgt analog der in der Kosmetik und Pharmazie üblichen Verfahren zur Herstellung von Hydrogelen. Eingesetzt werden dabei die üblichen Gelbildner. Erfindungsgemäß bevorzugt werden dabei Polyacrylsäure und ihre Derivate als Gelbildner eingesetzt.

Erfindungsgemäß vorteilhafte Polyacrylate sind Acrylat-Alkylacrylat-Copolymere, insbesondere solche, die aus der Gruppe der so genannten Carbomere oder Carbopole (Carbopol® ist eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

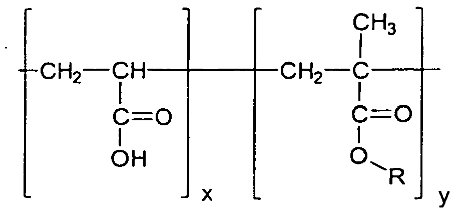

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind, bevorzugt Polyacrylate aus der Gruppe der Carbopole der Typen 980, 981, 1382, 2984, 5984 sowie besonders bevorzugt Carbomer 2001.

Ferner vorteilhaft sind Copolymere aus C₁₀₋₃₀-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Als hydrophile Gele werden bevorzugt Gele auf Basis von Polyacrylsäure und/oder deren Derivate, wie Alkali- und Erdalkalisalze, mit Aluminium-Ionen Vorvernetzte Polyacrylsäuren, oder Copolymerisate mit polyfunktionellen Monomeren gewählt.

Erfindungsgemäß vorteilhafte Hydrogele umfassen 2 bis 50 Gew.%, bevorzugt 5 bis 30 Gew.%, hydrophilen Gelbildner, bevorzugt Polyacrylsäure, und ca. 50 bis 98 Gew.%, bevorzugt 60 bis 95 Gew.% Wasser, jeweils bezogen auf die Gesamtmasse des hydrophilen Gels. In der finalen Gelkomposition wir das Hydrogel bevorzugt zu 15 bis 55 Gew.% eingesetzt, besonders bevorzugt im Bereich von 25 bis 45 Gew.%.

Die für eine erfindungsgemäße finale Gelkomposition eingesetzten Hydrogele können dabei beispielsweise eine komplexe dynamische Viskosität von 500 bis 5 000 Pa*s (25° C; 1 rad/s) aufweisen.

Bevorzugt umfasst die erfindungsgemäße Zubereitung
- ein hydrophobes Gel, umfassend maximal 50 Gew.% Polyisobutylen, bezogen auf die Gesamtmasse des hydrophoben Gels, und Parrafinöl und Vaseline als Mineralfette bzw. öle und
- ein hydrophiles Gel auf Basis von Polyacrylsäure, wobei die hydrophilen und hydrophoben Gele homogen verteilt sind.

Mit einer erfindungsgemäßen filmbildenden Gelkomposition zur Wund- und Hautbehandlung lassen sich die Vorteile der bekannten Systeme halbfester Zubereitungen (Pflaster und Creme) kombinieren und die deren jeweiligen Nachteile vermeiden helfen.

Bevorzugt umfast die erfindungsgemäße Zubereitung eine homogene Mischung aus hydrophilem Gel umfassend Polyacrylsäuregel/Wasser und hydrophoben Gel umfassend Polyisobutylen/Paraffinöl/Vaseline.

Mit den jeweiligen Anteilen an den unterschiedlichen Polymergelen wie auch deren jeweiliger eigener prozentualer wie chemischer Zusammensetzung, wie Kettenlängen, funktionelle Seitengruppen, Quervernetzung, kann gezielt das Wasseraufnahmevermögen bzw. die Wasserdampfdurchlässigkeit der Gelkomposition eingestellt werden. Damit lassen sich vorteilhafte Wundbehandlungsprodukte, wie insbesondere zur verbesserten feuchten Wundheilung, als auch Hautpflegeprodukte herstellen und anbieten.

Aufgrund der Kombination von hydrophoben und hydrophilen Polymergelen lassen sich sowohl hydrophile als auch hydrophobe Hilfs- oder Zusatzstoffe bzw. Wirkstoffe einzeln und in Kombination in eine einzige Gelkomposition einarbeiten.

Zur Optimierung der haptischen oder organoleptischen Eigenschaften des finalen Endproduktes können hydrophile wie hydrophobe Additive jedweder Art eingearbeitet werden.

Die erfindungsgemäßen kosmetischen und dermatologischen Zubereitungen können daher kosmetische Hilfsstoffe enthalten, wie sie üblicherweise in solchen Zubereitungen verwendet werden, z.B. Konservierungsmittel, Bakterizide, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Füllstoffe, Elektrolyte, organische Lösemittel oder Silikonderivate.

Als Wirkstoffe werden bevorzugt Folsäure und/oder deren Derivate eingesetzt.

Ebenso bevorzugt werden Wundheilsubstanzen wie Dexpanthenol und/oder auf Pflanzenextrakten basierende Wundheilsubstanzen wie Equisetum arvense, Aloe barbadensis, Arnica montana, Arnica chamissonis, Symphytum officinale, Solanum dulcamara, Echinacea pallida, Potentilla erecta, Trigonella foenum-graecum, Juglans regia, Linum usitatissimum, Terminalia sericea, Oenothera biennis, Centella asiatica, Arctium lappa, Capsella bursapastoris, Hypericum perforatum, Matricaria recutita, Chamomille recutita, Agrimonia eupatoria, Centaurea cyanus, Larrea tridentata, Populus spec., Echinacea pupurea, Calendula officinalis, Aesculus hippocastanum, Salvia officinalis, Plantago lanceolata, Quercus robur, Glycyrhiza glabra, Quercus petraea, Hamamelis virgian, Cardiospermum halicacabum,Betula, Urtica dioica, Buxus chinensis, Lavandula angustifolia, Lavandula hybrida, Crocus sativus, Smilax aspera, Melaleuca alternifolia, Aminosäuren oder Viola tricolor oder deren Salze oder Derivate oder Mischungen aus mindestens zwei davon erfindungsgemäß eingesetzt.

Ferner kommen als weitere Wundheilsubstanzen oder Hautpflegemittel Vitamine und der gleichen wie Glukosamin Sulfate Allantoin, Biotin, Chondroitin Sulfate, Coenzym Q10, Ascorbinsäure, Dexpanthenol, Honig/Honigextrakt, Niacinamid, Propolis, Vitamin A oder seine Ester, Vitamin C und seine Ester, Vitamin E und seine Ester oder deren Salze oder Derivate oder Mischungen aus mindestens zwei davon erfindungsgemäß in Betracht.

Erfindungsgemäß bevorzugt handelt es sich bei Wundheilsubstanzen um Dexpanthenol oder Extrakte der Ringelblume, vorzugsweise Calendulaöl; der Hamamelis, vorzugsweise D-Hamelose; oder der Kamille, vorzugsweise das Öl der Kamillenblüte - vorzugsweise Bisbolol oder Azulen - oder Mischungen aus mindestens zwei aller vorstehenden Substanzen.

Weiterhin ist es bevorzugt, dass jeweils eine der vorstehenden Wundheilsubstanzen in einer Mischung als Hauptkomponente vorliegen kann, wobei diese Hauptkomponente vorzugsweise mindestens 50 Gew.-%, vorzugsweise mindestens 70 Gew.-% und besonders bevorzugt mindestens 95 Gew.-%, jeweils bezogen auf die Mischung, vorliegen kann.

Als hautpflegende Substanzen werden bevorzugt Vitamine, Antioxidantien, Lichtschutzmittel, Insektenrepellentien, ätherische Öle, antimikrobielle Mittel, Moisturizer, Parfume und insbesonder Coenzym Q10, Ascorbinsäure und/oder Folsäure gewählt.

Durch die Zugabe von Wirkstoffen kommt es neben dem bei Anwendung aller wirkstoffhaltigen Zubereitungen vorhandenen Freisetzungsgradienten Haut/Vehikel erfindungsgemäß zu einem weiteren die Wirkstofffreisetzung positiv beeinflussenden dynamischen Konzentrationsgradienten innerhalb der Matrix. Dies dadurch, dass je nach Polarität des Wirkstoffs dieser in der hydrophoben oder hydrophilen Phase vorhanden ist und durch den zuvor beschriebenen Trocknungsprozess nach der Auftragung sich dieser Gradient verschiebt.

Grundlegend ist die Freisetzung eines Wirkstoffes aus einer Matrix umso besser, je schlechter die Löslichkeit des Wirkstoffs in der Matrix ist. Wird eine erfindungsgemäße Gelkomposition zur Versorgung einer offenen Wunde eingesetzt, so kommt es zunächst zur Aufnahme von Wundsekret, Blut etc. durch das in der Gelkomposition vorhandene hydrophile Gel. Dadurch steigt die Polarität innerhalb der Gelkomposition an und die Löslichkeit eines lipophilen Wirkstoffs in der Matrix wird herabgesetzt, d.h. die Freisetzung des lipophilen Wirkstoffs positiv beeinflusst.

Ist die Wunde geschlossen so dass kein Wundsekret mehr durch die Gelkomposition aufgenommen werden kann, bzw. bei Anwendung der Gelkomposition auf intakter Haut, kommt es zur Abgabe des in der Gelkomposition vorhandenen Wassers durch Verdunstung. Dadurch sinkt die Polarität innerhalb der Gelkomposition und die Löslichkeit eines hydrophilen Wirkstoffs in der Matrix wird herabgesetzt, d.h. die Freisetzung des hydrophilen Wirkstoff positiv beeinflusst.

Hergestellt wird eine erfindungsgemäße Gelkomposition in dem zunächst das hydrophobe Polymergel durch Quellung eines bevorzugt thermoplastischen Polymers in einem geeigneten Fettkörper oder einer Kombination geeigneter Fettkörper gefertigt wird. Erfindungsgemäß besonders geeignet als thermoplastische Polymere sind Polyisobutylene (PIB), als Fettkörper zur Quellung des PIBs Vaseline oder Paraffinöle oder Mischungen aus Vaseline oder Paraffinölen.

Über den Anteil und die Kettenlänge des eingesetzten PIBs, ggf. Mischungen von PIBs unterschiedlicher Kettenlänge, können die grundlegenden Eigenschaften des bei Anwendung resultierenden Films der Gelkomposition beeinflusst werden.

Erfindungsgemäß bevorzugte Polyisobutylene sind hochmolekulares PIB und/oder niedermolekulares PIB wie zuvor definiert.

Bevorzugt wird das thermoplastische Polymergel in Vaseline oder Paraffinöl gequollen, besonders bevorzugt in Mischungen von Vaseline und Paraffinöl. Die bevorzugten Verhältnisse zwischen Vaseline und Paraffinöl betragen dabei von 1 : 5 bis 5 : 1, insbesondere im Bereich von ca. 1 zu 1, was den bevorzugten Bereich 1: 1,2 mit umfasst.

Die Gesamtmenge des hydrophoben Polymergels in der finalen Gelkomposition kann dabei zwischen 10 und 90 Gew.% betragen. Bevorzugt zwischen 20 und 70 Gew.%, besonders bevorzugt zwischen 25 und 45 Gew.%, bezogen af die Gesamtmasse der Zubereitung.

Zur Herstellung der finalen filmbildenden Gelkomposition zur Wund- bzw. Hautbehandlung wird ein vorstehend beschriebenes Hydrogel in ein vorstehend beschriebenes hydrophobes Polymergel eingearbeitet. Die Einarbeitung erfolgt lösungsmittelfrei mittels handelsüblicher Kneter oder auch im Extruder bei Temperaturen von RT bis 90 °C.

Nachfolgend Beispielrezepturen stellen bevorzugte Zubereitungen dar. Die Anteile sind in Gewichtsprozent in Bezug auf die Gesamtmasse der filmbildende Gelkomposition wiedergegeben.

| Beispiel | I | II | III | IV | V | VI | VII | VIII | IX |
|---|---|---|---|---|---|---|---|---|---|
| Polyisobutylen B 12 | ----- | ----- | ----- | ----- | 12,04 | 12,04 | 10,00 | 17,50 | 9,82 |
| Polyisobutylen B 80 | 14,70 | 12,30 | 13,33 | 16,40 | 12,04 | 12,04 | ----- | ----- | ----- |
| Polyisobutylen B 150 | ----- | ----- | ----- | ----- | ----- | ----- | 15,00 | 7,50 | ----- |
| Vector 4114 | ----- | ----- | ----- | ----- | ----- | ----- | ----- | ----- | 8,05 |
| Vaseline | 12,30 | 10,30 | 11,12 | 14,18 | 12,04 | 12,04 | 30,00 | ----- | 12,16 |
| Paraffinöl | 14,90 | 12,40 | 13,45 | 16,15 | 14,70 | 14,70 | ----- | 30,00 | 14,87 |
| Wasser | 30,57 | 32,70 | 31,57 | 30,17 | 28,68 | 27,18 | 34,70 | 34,70 | 27,57 |
| Polyacrylsäure | 3,00 | 4,00 | 3,00 | 5,00 | 5,00 | 7,00 | 10,00 | 10,00 | 3,00 |
| NaOH | 0,15 | 0,30 | 0,15 | 0,10 | ----- | ----- | 0,30 | 0,30 | 0,15 |
| Bienenwachs | 7,88 | 9,00 | 5,88 | 4,50 | ----- | ----- | ----- | ----- | 7,88 |
| Glycerin | 12,50 | 15,00 | 9,50 | 11,00 | 11,00 | ----- | ----- | ----- | 16,50 |
| Polyvinylalkohol, teilverseift | 4,00 | 4,00 | 12,00 | ----- | ----- | ----- | ----- | ----- | ----- |
| Polyvinylpyrrolidon | ----- | ----- | ----- | 2,50 | 2,50 | ----- | ----- | ----- | ----- |
| Propylenglycollaurat | ----- | ----- | ----- | ----- | 2,00 | 15,00 | ----- | ----- | ----- |

Bei den Polyisobutylenen B12, B80 und B150 handelt es sich um Polyisobutylene mit unterschiedlichen Molmassen. Die Molmasse nimmt mit steigender Zahl zu. So hat beispielsweise B12 eine mittlere Molmasse von 55.000 Da, B80 von 800.000 Da und B150 von 2.600.000 Da.

Nachfolgend sind einige Untersuchungen der erfindungsgemäßen Zubereitungen im Vergleich mit unterschiedlichen Salben/Cremes hinsichtlich Wasserdampfdurchlässigkeit, Wasseraufnahmevermögen und Formstabilität der Zubereitungen dargelegt. Die erfindungsgemäßen filmbildenden Gelkompositionen zeigen dabei deutlich ihre hervorragende Eignung als Matrixsysteme für eine Applikation zur Förderung der feuchten Wundheilung und Hautpflege

**Tabelle 1: Zusammensetzung erfindungsgemäße Zubereitungen (Gew.%)**

| | Wö 04/04 | Wö 05/04 |
|---|---|---|
| Polyacrylsäure | 10,00 | 7,50 |
| Paraffinöl | 10,00 | 7,50 |
| Polyisobutylen | 25,00 | 18,75 |
| Vaseline | 40,00 | 30,00 |
| Wasser | 15,00 | 36,25 |

**Tabelle 2: Testergebnisse Wundheilsalben vs. erfindungsgemäße Zubereitungen**

| Nr. | Salbe | WDD (g/m²x 24 h)*) | Flüssigkeitsaufnahme nach 24 h (g/g und g/cm²) | Stabilität nach 24 h Flüssigkeitskontakt | Bemerkungen |
|---|---|---|---|---|---|
| 1 | Aquaphor Original Ointment | 30 - 50 | 0,04 - 0,05 / -0,004 | formstabil | |
| 2 | Eucerin 20 % Omega Fettsalbe | ∼7 | 0, 09 / -- | formstabil (nach 1 h) | |
| 3 | Bepanthen Wund- und Heilsalbe (Roche) | 8 | 0,05 / -- | formstabil (nach 1 h) | |
| 4 | Repithel Hydrogel (Mundipharm) | ± 0 | nicht bestimmbar | löst sich teilweise auf (nach 1 h) | ein amorphes Hydrogel mit 91 % Wassergehalt |
| 5 | Aquaphor + 5 % Elcema P050 | ∼ 30 | 0,04 / -- | nicht bestimmbar | |
| 6 | pH5 Eucerin Lotion F | ∼ 50 | n. b. | löst sich nicht, zerfließt aber | |
| 7 | Eucerin Reife Haut Vital Active Body | ∼ 550 | nicht bestimmbar | wird oberflächlich angelöst | |
| 8 | Eucerin Reinigungsemulsion | ∼ 280 | nicht bestimmbar | löst sich auf | |
| 9 | Wö 04/04 (Prototyp) | ∼ 6 | 1,85 / 0,13 | löst sich nicht auf | Bildet Film |
| 10 | Wö 05/04 (Prototyp) | ∼ 107 | 2,04 / 0,17 | löst sich nicht auf | Bildet Film |

| | | | | | |
|---|---|---|---|---|---|
| *) Differenz zwischen WDD mit Wasser und WDD ohne Wasser im Cup (um Abdampfeffekte bei wasserhaltigen Materialien mit zu berücksichtigen); Salben ca. 1 mm dick auf Trägermaterial Scar I oder Rayophane. Die Zubereitungen 1 bis 8 der Tabelle 2 stellen käuflich erwerbbare Hautpflege- oder Wundheilungsprodukte dar. | | | | | |

Die Wasserdampfdurchlässigkeit (WDD) und das (Wasser-) Flüssigkeitsaufnahmevermögen sind die bestimmenden Kriterien zur Eignung eines Wundverbandes für eine feuchte Wundheilung. Im Gegensatz zur trockenen Wundheilung, bei der die Wunde lediglich mit einem stark saugenden Material abgedeckt und gegen äussere Infektionsherde geschützt wird, kann durch feuchte Wundheilung der Heilungsprozess beschleunigt und gleichfalls ein besseres kosmetisches Ergebnis der Wundheilung erzielt werden. Eine zu starke Feuchtigkeit / Nässe wirkt sich wiederum negativ auf den Heilungsprozess aus. Daher ist es optimal, wenn das Wasseraufnahmevermögen und die Wasserdampfdurchlässigkeit eines Wundversorgungsproduktes entsprechend eingestellt werden können ohne dass das Produkt seine Formstabilität verliert, bzw. sich im Falle einer Salbe / eines Gel mit der Zeit auf der Wunde auflöst oder wegfliest.

Zur Messung der WDD werden in der Regel auf das jeweilige Produkt nötigenfalls abgewandelte Methoden aus DIN EN 13726-2 (Prüfverfahren für primäre Verbandsstoffe (Wundauflagen), Teil 2: Feuchtigkeitsdurchdringungsrate durchlässiger Folienverbände) oder ASTM E 96-95 (Standard Test Methods for Water Vapor Transmission of Materials) angewendet. Bei beiden Standards handelt es sich um sogenannte "gravimetrische Cup-Methoden". Hierbei werden Bechergefäße mit definierter Menge an Wasser gefüllt, diese mit dem Prüfmaterial abgedeckt (im Falle von streichfähigen Proben wie Salben / Gele werden diese definiert auf ein Substrat bekannter hoher WDD zur Abdeckung des Messgefäßes aufgetragen), das Messgefäß in einen Klimaschrank mit konstanter Temperatur und Luftfeuchtigkeit gestellt und nach definierter Zeit der Verlust an Wasser im Messgefäß bestimmt.

Weiterhin wird an der Probe das Wasseraufnahmevermögen gravimetrisch und die Formstabilität visuell bestimmt.

Wie Tabelle 2 zeigt nehmen hydrophobe Wundsalben auf Fettbasis, Zubereitungen 1 - 3, selbst nach Zusatz von 5% Cellulosederivaten (Elcema P050), Zubereitung 5, keine bis nur geringe Mengen an Wasser bzw. Wundexsudat auf und zeigen eine für feuchte Wundheilung zu geringe Wasserdampfdurchlässigkeit auf.

Andererseits bleiben diese Salben bei Wasserkontakt auch über längere Zeit formstabil und lösen sich nicht auf.

Emulsionen, Zubereitungen 6 - 8, zeigen abhängig vom Emulsionstyp O/W oder W/O mittlere bis gute Wasserdampfdurchlässigkeiten, nehmen dafür aber keine Feuchtigkeit formstabil auf sondern lösen sich auf bzw. zerfliessen dabei.

Für ein reines Hydrogel zur Wundbehandlung, Zubereitung 4, konnte weder die Wasserdampfdurchlässigkeit noch die Flüssigkeitsaufnahme bestimmt werden, da das Hydrogel sich bei dem Versuch auflöste.

Keine der Zubereitungen 1 bis 8 bildete einen Film, der im Ganzen von der Haut wieder entfernbar war.

Erfindungsgemäße Gelkompositionen, Zubereitungen 9 und 10, zeigten eine gute Flüssigkeitsaufnahme ohne ihre Formstabilität dabei zu verlieren.

Weiterhin zeigt Tabelle 2, dass die Wasserdampfdurchlässigkeit und die Flüssigkeitsaufnahme bei analogen sonstigen Eigenschaften der erfindungsgemäßen Zubereitungen (s. Tabelle 1) durch die Relationen von hydrophilem zu hydrophobem Gel sowie der Relationen der jeweiligen Gelkomponenten in sich, deutlich verändert werden kann. Zubereitung 10 (Wö 05/04) zeigt dadurch gegenüber Zubereitung 9 (Wö 04/04) eine um rund eine Zehnerpotenz höhere Wasserdampfdurchlässigkeit.

Dies ermöglicht die Herstellung individueller Wundversorgungsprodukte wie z.B. zur Behandlung kleinerer Schnitt-, Brand- oder Risswunden, offener Blasen, großflächiger Schürfwunden etc., wie auch dermatologischer Anwendungen, insbesondere als Kratz- und Scheuerschutz bei Neurodermitis, Hautflechten, Gürtelrose etc., oder auch rein kosmetische Anwendungen zur Faltenreduktion, Cellulitereduktion, Behandlung von Pigmentstörungen, Altersflecken etc. sind möglich und bevorzugt.

## Patentansprüche

1. Filmbildende Zubereitung umfassend mindestens ein hydrophobes Gel und mindestens ein hydrophiles Gel, **dadurch gekennzeichnet, dass** das hydrophobe Gel mindestens ein hydrophobes Polymer und ein oder mehrere Mineralfetten und/oder -öle umfasst und das hydrophile Gel im hydrophoben Gel homogen verteilt vorliegt.

2. Zubereitung nach Anspruch 1, **dadurch gekennzeichnet, dass** das hydrophile Gel mit einer Tröpfchengröße von 0,2 - 20 µm im hydrophoben Gel verteilt vorliegt.

3. Zubereitung nach einem der vorstehenden Ansprüche, **dadurch gekennzeichnet, dass** das hydrophobe Polymer zu einem Anteil von maximal 60 Gew.%, bezogen auf die Gesamtmasse des sich bildenden hydrophoben Gels, enthalten ist.

4. Zubereitung nach Anspruch 3, **dadurch gekennzeichnet, dass** das hydrophobe Polymer zu einem Anteil von maximal 50 Gel.%, bezogen auf die Gesamtmasse des sich bildenden hydrophoben Gels, enthalten ist.

5. Zubereitung nach Anspruch 3, **dadurch gekennzeichnet, dass** das hydrophobe Polymer zu einem Anteil von maximal 40 Gew.%, bezogen auf die Gesamtmasse des sich bildenden hydrophoben Gels, enthalten ist.

6. Zubereitung nach einem der vorstehenden Ansprüche, **dadurch gekennzeichnet, dass** als hydrophobes Polymer Polyisobutylen gewählt wird.

7. Zubereitung nach einem der vorstehenden Ansprüche, **dadurch gekennzeichnet, dass** als hydrophobes Polymer eine Mischung aus Polyisobutylen und anderen Polymeren, bevorzugt Styrol-Isopren-Styrol-Blockcopolymere (SIS), gewählt wird.

8. Zubereitung nach einem der vorstehenden Ansprüche, **dadurch gekennzeichnet, dass** das hydrophobe Gel eine Viskosität von 10 000 - 25 000 Pa*s (25° C; 1 rad/s) aufweist.

9. Zubereitung nach einem der vorstehenden Ansprüche, **dadurch gekennzeichnet, dass** als Mineralfette oder -öle ein oder mehrere Fette oder Öle gewählt aus der Gruppe Paraffinöle, Paraffinfette und Paraffinwachse gewählt werden.

10. Zubereitung nach Anspruch 9, **dadurch gekennzeichnet, dass** als Paraffinfett Vaseline gewählt wird.

11. Zubereitung nach Anspruch 9, **dadurch gekennzeichnet, dass** als Mineralfette oder -öle Paraffinöl und Vaseline gewählt werden.

12. Zubereitung nach Anspruch 11, **dadurch gekennzeichnet, dass** Paraffinöl und Vaseline in einem Gewichtsverhältnis von Vaseline zu Paraffinöl von 1 : 5 bis 5 : 1, gewählt wird.

13. Zubereitung nach einem der vorstehenden Ansprüche, **dadurch gekennzeichnet, dass** als hydrophile Gele Gele auf Basis von Polyacrylsaure und/oder deren Derivate gewählt werden.

14. Zubereitung nach Anspruch 13, **dadurch gekennzeichnet, dass** als Polyacrylsäurederivate Alkali- und Erdalkalisalze, mit Aluminium-Ionen Vorvernetzte Polyacrylsäuren, oder Copolymerisate mit polyfunktionellen Monomeren gewählt werden.

15. Zubereitung nach einem der vorstehenden Ansprüche umfassend
a. ein hydrophobes Gel, umfassend maximal 50 Gew.% Polyisobutylen, bezogen auf die Gesamtmasse des hydrophoben Gels, und Parrafinöl und Vaseline als Mineralfette bzw. öle und
b. ein hydrophiles Gel auf Basis von Polyacrylsäure, **dadurch gekennzeichnet, dass** die hydrophilen und hydrophoben Gele homogen verteilt sind.

16. Zubereitung nach einem der vorstehenden Ansprüche umfassend zusätzlich Lösungsvermittler, Feuchthaltemittel, Weichmacher, Aromastoffe, Färbemittel und/oder kosmetische und/oder pharmazeutische Wirkstoffe.

17. Verwendung einer Zubereitung nach einem der vorstehenden Ansprüche als Kosmetikum in der Hautpflege.

18. Verwendung einer Zubereitung nach einem der vorstehenden Ansprüche zur Herstellung eines Wundheilungsproduktes.

19. Hautpflege - und/oder Wundheilungsprodukt umfassend eine Zubereitung nach einem der vorstehenden Ansprüche 1 bis 16.

## Claims

1. Film-forming preparation comprising at least one hydrophobic gel and at least one hydrophilic gel, **characterized in that** the hydrophobic gel comprises at least one hydrophobic polymer and one or more mineral fats and/or oils, and the hydrophilic gel is present in the hydrophobic gel in homogeneously distributed form.

2. Preparation according to Claim 1, **characterized in that** the hydrophilic gel with a droplet size of 0.2-20 µm is present in the hydrophobic gel in distributed form.

3. Preparation according to one of the preceding claims, **characterized in that** the hydrophobic polymer is present to a fraction of at most 60% by weight, based on the total mass of the hydrophobic gel which is formed.

4. Preparation according to Claim 3, **characterized in that** the hydrophobic polymer is present to a fraction of at most 50% by weight, based on the total mass of the hydrophobic gel which is formed.

5. Preparation according to Claim 3, **characterized in that** the hydrophobic polymer is present to a fraction of at most 40% by weight, based on the total mass of the hydrophobic gel which is formed.

6. Preparation according to one of the preceding claims, **characterized in that** polyisobutylene is selected as a hydrophobic polymer.

7. Preparation according to one of the preceding claims, **characterized in that** a mixture of polyisobutylene and other polymers, preferably styreneisoprene-styrene block copolymers (SIS), is selected as hydrophobic polymer.

8. Preparation according to one of the preceding claims, **characterized in that** the hydrophobic gel has a viscosity of 10 000-25 000 Pa*s (25°C; 1 rad/s).

9. Preparation according to one of the preceding claims, **characterized in that** one or more fats or oils selected from the group of paraffin oils, paraffin fats and paraffin waxes are selected as mineral fats or oils.

10. Preparation according to Claim 9, **characterized in that** vaseline is selected as paraffin fat.

11. Preparation according to Claim 9, **characterized in that** paraffin oil and vaseline are selected as mineral fats or oils.

12. Preparation according to Claim 11, **characterized in that** paraffin oil and vaseline in a weight ratio of vaseline to paraffin oil of from 1:5 to 5:1 is selected.

13. Preparation according to one of the preceding claims, **characterized in that** gels based on polyacrylic acid and/or their derivatives are selected as hydrophilic gels.

14. Preparation according to Claim 13, **characterized in that** alkali metal salts and alkaline earth metal salts, polyacrylic acids pre-crosslinked with aluminium ions, or copolymers with polyfunctional monomers are selected as polyacrylic acid derivatives.

15. Preparation according to one of the preceding claims, comprising
a. a hydrophobic gel comprising at most 50% by weight of polyisobutylene, based on the total mass of the hydrophobic gel, and paraffin oil and vaseline as mineral fats or oils and
b. a hydrophilic gel based on polyacrylic acid, **characterized in that** the hydrophilic and hydrophobic gels are in homogeneously distributed form.

16. Preparation according to one of the preceding claims, comprising additionally solubility promoter, humectant, softener, aroma substances, colorants and/or cosmetic and/or pharmaceutical active ingredients.

17. Use of a preparation according to one of the preceding claims as cosmetic in skin care.

18. Use of a preparation according to one of the preceding claims for producing a wound healing product.

19. Skin care and/or wound healing product comprising a preparation according to one of the preceding Claims 1 to 16.

## Revendications

1. Composition filmogène comprenant au moins un gel hydrophobe et au moins un gel hydrophile, **caractérisée en ce que** le gel hydrophobe comprend au moins un polymère hydrophobe et une ou plusieurs graisses et/ou huiles minérales et le gel hydrophile se trouve réparti de manière homogène dans le gel hydrophobe.

2. Composition selon la revendication 1, **caractérisée en ce que** le gel hydrophile se trouve réparti dans le gel hydrophobe avec une grosseur des gouttes de 0,2-20 µm.

3. Composition selon l'une quelconque des revendications précédentes, **caractérisée en ce que** le polymère hydrophobe est contenu en une proportion d'au maximum 60% en poids, par rapport à la masse totale du gel hydrophobe se formant.

4. Composition selon la revendication 3, **caractérisée en ce que** le polymère hydrophobe est contenu en une proportion d'au maximum 50% en poids, par rapport à la masse totale du gel hydrophobe se formant.

5. Composition selon la revendication 3, **caractérisée en ce que** le polymère hydrophobe est contenu en une proportion d'au maximum 40% en poids, par rapport à la masse totale du gel hydrophobe se formant.

6. Composition selon l'une quelconque des revendications précédentes, **caractérisée en ce que** le polyisobutylène est choisi comme polymère hydrophobe.

7. Composition selon l'une quelconque des revendications précédentes, **caractérisée en ce qu'**on choisit comme polymère hydrophobe un mélange de polyisobutylène et d'autres polymères, de préférence des copolymères à blocs de styrène-isoprène-styrène (SIS).

8. Composition selon l'une quelconque des revendications précédentes, **caractérisée en ce que** le gel hydrophobe présente une viscosité de 10 000-25 000 Pa.s (25°C ; 1 rad/s).

9. Composition selon l'une quelconque des revendications précédentes, **caractérisée en ce qu'**on choisit comme graisses ou huiles minérales une ou plusieurs huiles ou graisses du groupe des huiles de paraffine, des graisses de paraffine et des cires de paraffine.

10. Composition selon la revendication 9, **caractérisée en ce qu'**on choisit de la vaseline comme graisse de paraffine.

11. Composition selon la revendication 9, **caractérisée en ce qu'**on choisit comme graisses ou huiles minérales de l'huile de paraffine et de la vaseline.

12. Composition selon la revendication 11, **caractérisée en ce que** l'huile de paraffine et la vaseline sont choisies dans un rapport pondéral de vaseline à huile de paraffine de 1:5 à 5:1.

13. Composition selon l'une quelconque des revendications précédentes, **caractérisée en ce qu'**on choisit comme gels hydrophiles des gels à base de poly(acide acrylique) et/ou ses dérivés.

14. Composition selon la revendication 13, **caractérisée en ce qu'**on choisit comme dérivés de poly(acide acrylique) des sels de métal alcalin et alcalino-terreux, des poly(acides acryliques) préréticulés avec des ions d'aluminium ou des copolymères avec des monomères polyfonctionnels.

15. Composition selon l'une quelconque des revendications précédentes comprenant
a. un gel hydrophobe, comprenant au maximum 50% en poids de polyisobutylène, par rapport à la masse totale du gel hydrophobe et de l'huile de paraffine et de la vaseline comme huiles ou, selon le cas, graisses minérales et
b. un gel hydrophile à base de poly(acide acrylique) **caractérisée en ce que** les gels hydrophiles et hydrophobes sont répartis de manière homogène.

16. Composition selon l'une quelconque des revendications précédentes, comprenant en outre des promoteurs de solubilité, des humectants, des plastifiants, des parfums, des teintures et/ou des substances actives cosmétiques et/ou pharmaceutiques.

17. Utilisation d'une composition selon l'une quelconque des revendications précédentes comme cosmétique dans les soins de la peau.

18. Utilisation d'une composition selon l'une quelconque des revendications précédentes pour la préparation d'un produit cicatrisant.

19. Produit de soins de la peau et/ou cicatrisant, comprenant une composition selon l'une quelconque des revendications précédentes 1 à 16.
